(19) Europäisches Patentamt
European Patent Office
Office européen des brevets

(11) **EP 4 702 972 A1**

(12) **EUROPEAN PATENT APPLICATION**

(43) Date of publication:
04.03.2026 Bulletin 2026/10

(21) Application number: 25195007.7

(22) Date of filing: 08.08.2025

(51) International Patent Classification (IPC):
*A61K 9/06* (2006.01)  *A61K 47/10* (2017.01)
*A61K 47/36* (2006.01)  *A61K 31/573* (2006.01)
*A61K 31/58* (2006.01)  *A61P 19/02* (2006.01)

(52) Cooperative Patent Classification (CPC):
A61K 9/06; A61K 9/0019; A61K 31/573;
A61K 31/58; A61K 47/10; A61K 47/36; A61P 19/02

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 30.08.2024 TW 113132817

(71) Applicant: Scivision Biotech Inc.
Kaohsiung City 806 (TW)

(72) Inventors:
• HAN, TAI-SHIEN
806 KAOHSIUNG CITY (TW)

• PAN, TSUNG-WEI
806 KAOHSIUNG CITY (TW)
• CHEN, TOR-CHERN
806 KAOHSIUNG CITY (TW)
• CHEN, CHUN-CHANG
806 KAOHSIUNG CITY (TW)
• YEN, WEI-HSUAN
806 KAOHSIUNG CITY (TW)
• KAO, MING-HSIEN
806 KAOHSIUNG CITY (TW)

(74) Representative: 2K Patentanwälte Blasberg
Kewitz & Reichel
Partnerschaft mbB
Schumannstrasse 27
60325 Frankfurt am Main (DE)

(54) **METHOD FOR MANUFACTURING CROSSLINKED HYALURONIC ACID GEL CONTAINING TRIAMCINOLONE AND CROSSLINKED HYALURONIC ACID GEL**

(57) The present disclosure provides a method for manufacturing a crosslinked hyaluronic acid gel and a crosslinked hyaluronic acid gel. The crosslinked hyaluronic acid gel contains substantially evenly distributed triamcinolone.

Figure 1

EP 4 702 972 A1

**Description**

FIELD OF THE INVENTION

The present disclosure relates to a method for manufacturing a crosslinked hyaluronic acid gel and the crosslinked hyaluronic acid gel, particularly a crosslinked hyaluronic acid gel containing triamcinolone.

BACKGROUND OF THE INVENTION

Osteoarthritis (OA), also known as degenerative arthritis, is usually caused by long-term uneven stress on a joint, leading to degeneration and wear of the articular cartilage. This results in repeated inflammation or effusion, further causing the formation of bone spurs, joint deformity, and loss of elasticity, which in turn leads to joint pain, stiffness, and impaired mobility. Hyaluronic acid (HA) is the main component of synovial fluid in the joint cavity. It has high biocompatibility and low side effects. Injecting hyaluronic acid into the joint can lubricate the cartilage surface and improve joint mobility, showing efficacy for mild to moderate osteoarthritis. However, linear hyaluronic acid molecules are more easily absorbed and degraded by the human body. If cross-linked hyaluronic acid is used, its degradation time *in vivo* can be prolonged, and the therapeutic effect can last for more than six months.

There is an urgent need in the industry to improve the application of hyaluronic acid in the treatment of osteoarthritis.

SUMMARY OF THE INVENTION

The present disclosure provides a crosslinked hyaluronic acid gel containing triamcinolone and a method for manufacturing the same. The crosslinked hyaluronic acid gel exhibits a sustained-release effect for triamcinolone and can be used as an intra-articular injectable formulation.

Accordingly, the present disclosure provides a method for manufacturing a crosslinked hyaluronic acid gel, comprising steps of:

(a) mixing hyaluronic acid or a metal salt thereof and triamcinolone or a pharmaceutical acceptable salt or ester thereof to form a mixture;
(b) adding water into the mixture of step (a) to form a colloid; and
(c) crosslinking the colloid of step (b) with 1,4-butanediol diglycidyl ether (BDDE).

In some embodiments, triamcinolone or the pharmaceutical acceptable salt or ester thereof of step (a) is triamcinolone acetonide 21-oic acid methyl ester, triamcinolone benetonide, triamcinolone hexacetonide or triamcinolone diacetate. In another embodiment, triamcinolone or the pharmaceutical acceptable salt or ester thereof of step (a) is triamcinolone hexacetonide.

In some embodiments, in step (a), a weight ratio of hyaluronic acid or the metal salt thereof to triamcinolone or the pharmaceutical acceptable salt or ester thereof is about 10:1 to about 10:15.

In some embodiments, in step (a), hyaluronic acid or the metal salt thereof and/or triamcinolone or the pharmaceutical acceptable salt or ester thereof is in solid form.

In some embodiments, in step (a), hyaluronic acid or the metal salt thereof has a water content of about 20% or less and/or a particle size of 200 $\mu$m or less.

In some embodiments, in step (a), triamcinolone or the pharmaceutical acceptable salt or ester thereof has a water content of about 2% or less and/or a particle size of 5 $\mu$m or less.

In some embodiments, in step (b), a weight ratio of hyaluronic acid or the metal salt thereof to water is about 1:1 to about 1:6.

In some embodiments, in step (c), a reaction concentration of hyaluronic acid or the metal salt thereof is about 10 wt % to about 30 wt %.

In some embodiments, the method further comprises (d) steam sterilizing the crosslinked product of step (c).

The present disclosure further provides a crosslinked HA gel, comprising crosslinked hyaluronic acid or a metal salt thereof, and triamcinolone or a pharmaceutical acceptable salt or ester thereof, which is substantially evenly dispersed in the crosslinked hyaluronic acid gel, wherein the crosslinked hyaluronic acid gel is free of polysorbate.

In some embodiments, the crosslinked hyaluronic acid or a metal salt thereof is crosslinked with BDDE.

In some embodiments, a content of the crosslinked hyaluronic acid or a metal salt thereof is about 1.0 wt % to about 5.0 wt %.

In some embodiments, a content of triamcinolone or the pharmaceutical acceptable salt or ester thereof is about 0.1 wt % to about 5.0 wt %.

In some embodiments, a weight ratio of hyaluronic acid or the metal salt thereof to triamcinolone or the pharmaceutical acceptable salt or ester thereof is about 10:1 to about 10:15.

In some embodiments, the crosslinked HA gel has an average particle diameter of about 150 μm to about 1000 μm.

In some embodiments, the crosslinked HA gel has a modulus of elasticity (G') measured at 25 °C and 1 Hz of about 50 Pa to about 1500 Pa, and a modulus of viscosity (G'') measured at 25 °C and 1 Hz of about 10 Pa to about 150 Pa.

In some embodiments, the crosslinked HA gel further comprises a polyol. In one embodiment, the polyol is selected from the group consisting of mannitol and sorbitol.

In some embodiments, the crosslinked HA gel further comprises linear hyaluronic acid.

In some embodiments, the crosslinked HA gel further comprises auto-crosslinked hyaluronic acid.

In some embodiments, the crosslinked HA gel consists of the crosslinked hyaluronic acid or the metal salt thereof, triamcinolone or the pharmaceutical acceptable salt or ester thereof, optionally a polyol, optionally a linear hyaluronic acid, and, optionally an auto-crosslinked hyaluronic acid.

The present disclosure further provides a method for treating osteoarthritis in a subject, comprising administering the crosslinked HA gel to the subject. The present disclosure also provides the crosslinked hyaluronic acid gel for use in the treatment of osteoarthritis.

BRIEF DESCRIPTION OF DRAWINGS

FIG. 1 shows the sustained release experimental results of triamcinolone hexacetonide in the gels of Example 1, Example 2, Comparative Example 1, and the commercial product Cingal®.

FIG. 2 shows the modulus of elasticity (G') and modulus of viscosity (G'') values measured at different frequencies for the products of varying auto-crosslinked HA colloid contents in Example 6. Solid symbols represent the modulus of elasticity G' (•: auto-crosslinked HA colloid content 0 wt %, ■: auto-crosslinked HA colloid content 10 wt %, ▲: auto-crosslinked HA colloid content 30 wt %); open symbols represent the modulus of viscosity G'' (○: auto-crosslinked HA colloid content 0 wt %, □: auto-crosslinked HA colloid content 10 wt %, △: auto-crosslinked HA colloid content 30 wt %).

FIG. 3 shows the effect of shear rate on shear viscosity for the products with varying auto-crosslinked HA colloid contents in Example 6. •: auto-crosslinked HA colloid content 0 wt %, ■: auto-crosslinked HA colloid content 10 wt %, ▲: auto-crosslinked HA colloid content 30 wt %.

DETAILED DESCRIPTION OF THE INVENTION

Unless otherwise defined, all technical and scientific terms used herein have the same meaning as commonly understood by one of ordinary skill in the art to which this disclosure pertains. In the case of conflict, the present document, including definitions, will control.

As used in this specification and the appended claims, the singular forms "a," "an" and "the" include plural references unless the content clearly dictates otherwise.

As used in this specification and the appended claims, the term "or" means "and/or" unless the content clearly dictates otherwise. In the case of multiple dependent claims, the term "or" is intended to refer back to more than one of the foregoing independent or dependent claims by way of substitution only.

As used in this specification and the appended claims, the term "about" will be understood by those skilled in the art and will vary to some extent depending upon the context in which it is used. If the use of the term is not clear to one of ordinary skill in the art in the context in which it is used, "about" shall mean within 5%, 4%, 3%, 2%, or 1% of the specified term.

As used in this specification and the appended claims, the term "pharmaceutically acceptable salt" refers to a salt that retains the desired biological activity of the target compound and exhibits minimal undesired toxicological effects. Such pharmaceutically acceptable salts may be prepared in situ during the final isolation and purification of the compound, or by reacting the purified compound in its free acid or free base form with a suitable base or acid, respectively.

As used in this specification and the appended claims, the term "pharmaceutically acceptable ester" refers to an ester that retains the desired biological activity of the target compound and exhibits minimal undesired toxicological effects. For example, the "triamcinolone" described herein may form an ester via its alcohol functional group with one or more linear or branched carboxylic acids, wherein the one or more linear or branched carboxylic acids may each contain, for example, 2 to 12 carbon atoms.

Corticosteroids are steroid hormones produced and secreted by the adrenal cortex, and can also be synthesized artificially. Intra-articular injection of corticosteroids can provide short-term relief of osteoarthritis (OA) flare-ups by inhibiting cyclooxygenase-2 (COX-2), thereby achieving anti-inflammatory and analgesic effects.

In general, intra-articular injection of corticosteroids can relieve pain in OA patients for up to 4 weeks, while intra-articular injection of hyaluronic acid has an even better effect, lasting 8 to 26 weeks. Corticosteroids provide rapid and short-term pain relief within a few days after injection, whereas crosslinked hyaluronic acid offers long-term efficacy.

However, most corticosteroids are insoluble in water, so they are usually used as suspensions for OA treatment. To form a suspension, surfactants such as polysorbate 80 are typically added. Polysorbate 80 is a non-ionic surfactant containing ether bonds, which can undergo auto-oxidation, ethylene oxide subunit cleavage, and fatty acid ester bond hydrolysis in aqueous solution, forming formic acid, acetic acid, formaldehyde, and hydrogen peroxide, thereby affecting the stability of the product.

To address one or more of the foregoing issues, the present disclosure uses hyaluronic acid (HA) to replace surfactants such as polysorbate 80. By utilizing the viscosity of the HA solution, the suspension stability of corticosteroids in the formulation is enhanced. In another aspect, HA is further crosslinked to delay the absorption of crosslinked HA in the body, thereby achieving a sustained-release effect and prolonging the anti-inflammatory and analgesic efficacy of corticosteroids. However, during the crosslinking reaction of HA, due to the poor water solubility of corticosteroids, they tend to aggregate upon contact with the aqueous solvent, resulting in the formation of suspended matter on the surface of the solution and adhesion to the container surface. This leads to a decrease in the corticosteroid content in the product and uneven dispersion.

Accordingly, the present disclosure provides a method for manufacturing a crosslinked hyaluronic acid gel, comprising steps of:

> (a) mixing hyaluronic acid or a metal salt thereof and triamcinolone or a pharmaceutical acceptable salt or ester thereof to form a mixture;
> (b) adding water into the mixture of step (a) to form a colloid; and
> (c) crosslinking the colloid of step (b) with 1,4-butanediol diglycidyl ether (BDDE).

In an embodiment of the present disclosure, the corticosteroid powder is initially mixed with solid HA powder or filaments, and an aqueous solvent is then added to the mixture to allow HA to absorb the solvent and swell, forming a colloidal mixture. This ensures that the corticosteroid is evenly dispersed within the colloid and prevents it from adhering to the container. Subsequently, the remaining aqueous solvent, base, and crosslinking agent are added to conduct the crosslinking reaction. This allows the corticosteroid to be evenly dispersed within the crosslinked HA gel, which also provides a sustained-release function.

HA is a polysaccharide composed of repeating disaccharide units formed by $\beta$-1,3-N-acetyl glucosamine and $\beta$-1,4-glucuronic acid linked via $\beta$-1,4 bonds, with these disaccharide units further connected through $\beta$-1,3 linkages to form a high molecular weight linear polymer. HA commonly exists in the form of metal salts, such as potassium hyaluronate, lithium hyaluronate, and sodium hyaluronate, with sodium hyaluronate being preferred. There is no particular limitation on the average molecular weight of HA or the metal salt thereof; for example, it may range from about 100,000 to about 3,500,000, or from about 1,000,000 to about 1,500,000.

Triamcinolone is a corticosteroid that can be used to treat osteoarthritis and exhibits superior stability. Triamcinolone is available in various forms, including but not limited to triamcinolone acetonide 21-oic acid methyl ester, triamcinolone benetonide, triamcinolone hexacetonide (TH), and triamcinolone diacetate. Triamcinolone can be sterilized (simulated sterilization conditions: heating at 121°C for 15 to 30 minutes), and can maintain its chemical integrity after being stored at 80°C for 24 hours or at room temperature for 2 years.

There are theoretically no limitations on the mixing methods in the aforementioned steps (a), (b), and/or (c), as long as the purpose of mixing can be achieved. For example, mixing may be performed manually or by machine, including but not limited to stirring with a handheld stirring rod, or by using machines such as a kneader, mixer, or planetary mixer.

In some embodiments, the weight ratio of HA or the metal salt thereof to triamcinolone or the pharmaceutical acceptable salt or ester thereof in step (a) is about 10:1 to about 10:15, for example, 10:2, 10:3, 10:4, 10:5, 10:6, 10:7 or higher, and/or 10:15, 10:14, 10:13, 10:12, 10:11, 10:10, 10:9, 10:8 or lower. In one embodiment, depending on the application of the drug, the weight ratio of HA or the metal salt thereof to triamcinolone or the pharmaceutical acceptable salt or ester thereof is about 10:2 to about 10:10 or about 10:3 to about 10:10.

Generally, after absorbing water, HA or the metal salt thereof forms a colloid with extremely high viscosity, making it difficult to evenly mix the colloid with triamcinolone or the pharmaceutical acceptable salt or ester thereof. Therefore, in one specific embodiment of the present disclosure, HA or the metal salt thereof and triamcinolone or the pharmaceutical acceptable salt or ester thereof are initially mixed in dry or solid states, thereby allowing triamcinolone or the pharmaceutical acceptable salt or ester thereof to be evenly distributed in the mixture. While not bound by any theory, it is believed that due to the poor water solubility of triamcinolone or the pharmaceutical acceptable salt or ester thereof, it cannot be evenly dispersed in water. The method of the present disclosure utilizes the high viscosity of HA or the metal salt thereof after water absorption to enable triamcinolone or the pharmaceutical acceptable salt or ester thereof to be evenly dispersed in the colloid, thereby preventing aggregation of triamcinolone or the pharmaceutical acceptable salt or ester thereof.

The aforementioned "dry state" refers to a condition in which HA or the metal salt thereof and triamcinolone or the pharmaceutical acceptable salt or ester thereof are not dissolved in water nor have absorbed water to form a colloid, and are in a solid state with low water content (moisture content), such as in the form of powder, filament, or granule. That is, in certain embodiments, in step (a), HA or the metal salt thereof and/or triamcinolone or the pharmaceutical acceptable salt or ester thereof are in the form of powder, filament, and/or granule.

For example, HA or the metal salt thereof may be in a solid powder or filament form, such as using HA or the metal salt thereof in powder form. In certain embodiments, the water content of HA or the metal salt thereof in step (a) is less than about 20%, 18%, 16%, 14%, 12%, 10%, 8%, 6%, 4%, or 2%.

Alternatively, in certain specific embodiments of the present disclosure, the particle size of HA or the metal salt thereof is about 250 $\mu$m, 220 $\mu$m, 200 $\mu$m, 180 $\mu$m, 160 $\mu$m, 140 $\mu$m, 120 $\mu$m, 100 $\mu$m, 80 $\mu$m, 60 $\mu$m, 40 $\mu$m, or less than 20 $\mu$m. The aforementioned "particle size" may refer to the length of the longest side of the powder, filament, and/or granule, or may refer to the average particle diameter.

For example, triamcinolone or the pharmaceutical acceptable salt or ester thereof may be in a granular form, and in certain specific embodiments, in a powder form or as micronized particles. The particle size/diameter distribution of B may be about 1.0 $\mu$m to about 15.0 $\mu$m, about 2.0 $\mu$m to about 14.0 $\mu$m, about 3.0 $\mu$m to about 13.0 $\mu$m, about 4.0 $\mu$m to about 12.0 $\mu$m, about 5.0 $\mu$m to about 11.0 $\mu$m, about 6.0 $\mu$m to about 10.0 $\mu$m, or about 7.0 $\mu$m to about 9.0 $\mu$m; the average particle size/diameter may be about 1.0 $\mu$m to about 5.0 $\mu$m, about 1.0 $\mu$m to about 4.0 $\mu$m, or about 1.0 $\mu$m to about 3.0 $\mu$m. The average particle size/diameter of triamcinolone or the pharmaceutical acceptable salt or ester thereof used in the present invention can be measured using a laser particle size analyzer (PARTICA LA-960, HORIBA) at 25 °C, with normal saline containing 0.2% polysorbate 80 as the suspension solvent. In certain embodiments, the water content of triamcinolone or the pharmaceutical acceptable salt or ester thereof in step (a) is less than about 2%, 1.8%, 1.6%, 1.4%, 1.2%, 1.0%, 0.8%, 0.6%, or 0.4%.

In another aspect, in certain specific embodiments of the present disclosure, the particle size of triamcinolone or the pharmaceutical acceptable salt or ester thereof is about 5 $\mu$m, 4 $\mu$m, 3 $\mu$m, 2 $\mu$m, or less than 1 $\mu$m. The aforementioned "particle size" may refer to the length of the longest side of the powder, filament, and/or granule, or generally refers to the average particle diameter.

In some embodiments, the weight ratio of HA or the metal salt thereof to water in step (b) is about 1:1 to about 1:6, for example, 1:2, 1:3 or more, and/or 1:5, 1:4 or less; in one embodiment, about 1:2 to about 1:6. This allows the mixture to form a colloid with moderate viscosity, facilitating the subsequent crosslinking reaction.

The crosslinking reaction in step (c) may be conducted in a basic (alkaline) environment. In one specific embodiment of the present disclosure, the crosslinking reaction may be performed as described in U.S. Patent No. 9,371,402, the entirety of which is incorporated herein by reference into the present application. For example, a two-stage crosslinking reaction at different temperatures may be used, or a single-stage low-temperature reaction may be employed, and the resulting colloidal product does not require purification. Detailed reaction conditions, including but not limited to temperature and reaction time, are as described in U.S. Patent No. 9,371,402.

In certain embodiments, the reaction concentration of HA or the metal salt thereof in step (c) is about 10 wt % to about 30 wt %, for example, 12 wt %, 14 wt %, 16 wt %, 18 wt % or more, and/or 28 wt %, 26 wt %, 24 wt %, 22 wt %, 20 wt % or less; or about 12 wt % to about 15 wt %. Prior to the reaction, HA or the metal salt thereof must be maintained at a certain concentration to ensure sufficiently high viscosity to prevent aggregation of triamcinolone or the pharmaceutical acceptable salt or ester thereof. In addition, an appropriate reaction concentration can enhance the effectiveness of the crosslinking reaction. In one embodiment, the reaction concentration of HA or the metal salt thereof is about 10 wt % to about 20 wt %, about 11 wt % to about 19 wt %, about 12 wt % to about 18 wt %, about 13 wt % to about 17 wt %, about 14 wt % to about 16 wt %, or about 12 wt % to about 15 wt %.

While not willing to be bound by any theory, the HA or the metal salt thereof crosslinked with BDDE has a stronger crosslinking strength, which can prolong its degradation time *in vivo* and exhibits good biocompatibility. In some embodiments, the reaction concentration of triamcinolone or the pharmaceutical acceptable salt or ester thereof is about 0.05 wt % to about 2.0 wt %, for example, about 0.1 wt %, 0.3 wt %, 0.5 wt %, 0.7 wt %, 0.9 wt % or more, and/or 1.7 wt %, 1.5 wt %, 1.3 wt %, 1.0 wt % or less, about 0.1 wt % to about 1.0 wt %, or about 0.2 wt % to about 0.5 wt %.

The crosslinking reaction of step (c) is conducted in a basic (alkaline) environment. In some embodiments, a base (alkali) such as sodium hydroxide or potassium hydroxide is used to provide the basic (alkaline) environment, at a concentration of about 0.2 wt % to about 2.0 wt %, for example, about 0.5 wt %, 0.7 wt %, 0.9 wt % or more, and/or 1.7 wt %, 1.5 wt %, 1.3 wt %, 1.0 wt % or less, about 0.5 wt % to about 1.5 wt %, or about 0.6 wt % to about 1.0 wt %.

In some embodiments, the method further comprises (d) steam sterilizing the crosslinked product of step (c).

Steam sterilization refers to the use of high temperature and high pressure to generate saturated steam, thereby inactivating microorganisms. For example, a commonly used autoclave may be employed, performing steam sterilization at a temperature of about 121 °C to about 135 °C and a pressure of about 1.06 kg/cm$^2$ to about 2.1 kg/cm$^2$ for about 5

minutes to about 30 minutes.

In some embodiments, prior to steam sterilization, the colloidal product of the crosslinking reaction may be crushed into small pieces (e.g., granules, stripes or strings). The crushing method is not limited and may include the use of a metal sieve, blade, or extrusion device. Subsequently, depending on the intended use, the crushed colloid may be mixed with a buffer solution to allow swelling, and, if necessary, adjusted to near neutral pH (for example, about pH 6.5 to 7.8 or about pH 6.5 to 7.5), followed by homogenization. The buffer solution may be a phosphate buffer solution, with a concentration of about 1 mM to about 100 mM (for example, about 4 mM to about 100 mM, or about 4 mM to about 50 mM), and a pH of about 6.5 to about 7.8. The homogenized colloidal product may be filled into a suitable container (for example, a glass or plastic syringe) and then subjected to steam sterilization.

In some embodiments, a polyol, such as sorbitol, mannitol, etc., may be added to the colloidal product of the crosslinking reaction before steam sterilization. For example, the polyol may be added to a sodium phosphate buffer solution and then mixed with the crushed gel.

The present disclosure further provides a crosslinked HA gel, comprising crosslinked hyaluronic acid or a metal salt thereof, and triamcinolone or a pharmaceutical acceptable salt or ester thereof, which is substantially evenly dispersed in the crosslinked hyaluronic acid gel, wherein the crosslinked hyaluronic acid gel is free of polysorbate.

The disclosed crosslinked HA gel comprises triamcinolone or the pharmaceutical acceptable salt or ester thereof, which is substantially evenly dispersed, and exhibits a sustained-release effect for triamcinolone or the pharmaceutical acceptable salt or ester thereof. The crosslinked HA gel can be sterilized and does not contain easily degradable surfactants (e.g., polysorbates), thus is suitable for use as an intra-articular injection. The crosslinked HA gel may be formed by the aforementioned methods or may be prepared by other methods.

The term "substantially evenly dispersed" as used herein refers to triamcinolone or the pharmaceutical acceptable salt or ester thereof being dispersed within crosslinked HA gel such that aggregation or precipitation is not likely to occur. The dispersion evenness of triamcinolone or the pharmaceutical acceptable salt or ester thereof in the crosslinked HA gel can be evaluated by content analysis. For example, multiple samples may be collected from different locations within the crosslinked HA gel for content analysis (e.g., collecting 9 samples, with each sample measured 3 times), and the relative standard deviation (RSD) value obtained is used for assessment. The term "Substantially evenly dispersed" refers to an RSD value of about 5.0% or less, about 2.0% or less, or about 1.5% or less.

In certain embodiments, HA or the metal salt thereof is crosslinked with BDDE. Therefore, the structural strength of the crosslinked HA gel is stronger, which can prolong the degradation time *in vivo* and provides good biocompatibility.

In certain embodiments, the content of the crosslinked HA or the metal salt thereof is about 1.0 wt % to about 5.0 wt %, for example, about 1.5 wt %, 2.0 wt %, 2.5 wt %, 3 wt % or more, and/or 4.5 wt %, 4.0 wt %, 3.5 wt %, 3.0 wt % or less, about 1.0 wt % to about 3.0 wt %, or about 1.0 wt % to 2.0 wt %. The content of the crosslinked HA or the metal salt thereof in the gel can be determined by the carbazole assay as described in the European Pharmacopoeia (Ph. Eur.) 10.0.

In certain embodiments, the content of triamcinolone or the pharmaceutical acceptable salt or ester thereof is about 0.1 wt % to about 5.0 wt %, for example, about 0.5 wt %, 1.0 wt %, 1.5 wt %, 2.0 wt %, 2.5 wt % or more, and/or 4.5 wt %, 4.0 wt %, 3.5 wt %, 3.0 wt % or less, about 0.45 wt % to about 3.0 wt %, or about 0.6 wt % to about 1.2 wt %. The content analysis of triamcinolone or the pharmaceutical acceptable salt or ester thereof in the crosslinked HA gel may be performed by first hydrolyzing the crosslinked HA gel with hyaluronidase, and then analyzing the triamcinolone or the pharmaceutical acceptable salt or ester thereof content in the hydrolysate by high performance liquid chromatography (HPLC) according to the method described in the Ph. Eur. 10.0.

In certain embodiments, the weight ratio of the crosslinked HA or the metal salt thereof to triamcinolone or the pharmaceutical acceptable salt or ester thereof is about 10:1 to about 10:15, for example, 10:2, 10:3, 10:4, 10:5, 10:6, 10:7 or more, and/or 10:15, 10:14, 10:13, 10:12, 10:11, 10:10, 10:9, 10:8 or less. In one embodiment, depending on the application of the drug, the weight ratio of the crosslinked HA or the metal salt thereof to triamcinolone or the pharmaceutical acceptable salt is about 10:2 to about 10:10 or about 10:3 to about 10:10.

In certain embodiments, the average particle size of crosslinked HA gel is about 150 $\mu$m to about 1000 $\mu$m, about 200 $\mu$m to about 950 $\mu$m, about 250 $\mu$m to about 900 $\mu$m, about 300 $\mu$m to about 850 $\mu$m, about 350 $\mu$m to about 800 $\mu$m, about 400 $\mu$m to about 750 $\mu$m, about 450 $\mu$m to about 700 $\mu$m, about 500 $\mu$m to about 650 $\mu$m, or about 200 $\mu$m to about 800 $\mu$m. The average particle size is measured using physiological saline as the suspension solvent at 25°C, with a particle size analyzer (PARTICA LA-960, HORIBA). The aforementioned average particle size can be adjusted by reaction conditions or subsequent processes, such as changing the homogenization equipment and homogenization time, or using a sieve with a specific pore size or a screw extrusion granulator equipped with a sieve.

In certain embodiments, the viscoelastic properties of the crosslinked HA gel, measured at 25 °C and 1.0 Hz, are as follows: the modulus of elasticity (G') is about 50 Pa to about 1500 Pa, about 100 Pa to about 1100 Pa, about 150 Pa to about 1050 Pa, about 200 Pa to about 1000 Pa, about 250 Pa to about 950 Pa, about 300 Pa to about 900 Pa, about 350 Pa to about 850 Pa, about 400 Pa to about 800 Pa, about 450 Pa to about 750 Pa, about 500 Pa to about 700 Pa, or about 150 Pa to about 350 Pa; the modulus of viscosity (G'') under the same measurement condition is about 10 Pa to about 150 Pa, about 20 Pa to about 140 Pa, about 30 Pa to about 130 Pa, about 40 Pa to about 120 Pa, about 50 Pa to about 110 Pa, about 60 Pa to about 100 Pa, about 70 Pa to about 90 Pa, about 20 Pa to about 100 Pa, or about 20 Pa to about 60 Pa. The aforementioned viscoelastic properties (i.e., modulus of elasticity (G') and a modulus of viscosity (G")) can be measured using a rheometer (TA, AR2000ex) at 25 °C and 1.0 Hz with a 20 mm metal plate. The viscoelastic properties of the crosslinked HA gel can be adjusted by appropriate reaction conditions, such as the concentration of HA, the amount of crosslinking agent and base (alkali) added, reaction temperature, and reaction time.

In certain embodiments, the degree of crosslinking (CrD) of the crosslinked HA gel is about 0.05% to about 5.0%, preferably about 1.0% to about 3.0%, and more preferably about 1.0% to about 2.0%. The degree of crosslinking (CrD) relates to the conditions of the crosslinking reaction. Different degrees of crosslinking can be obtained by varying the reaction conditions, such as the concentration of the crosslinking agent, the concentration of HA, the concentration of the base, and the molecular weight of HA.

In certain embodiments, the crosslinked HA gel further comprises a polyol. In one embodiment, the polyol is selected from the group consisting of mannitol and sorbitol. The addition of polyol can increase resistance to free radical degradation, thereby reducing the degradation rate of the crosslinked HA or the metal salt thereof in the joint and enhancing its effect as a viscosupplementation. In certain embodiments, the concentration of polyol may be about 0.1 wt % to 10.0 wt %, for example, 0.5 wt %, 1.0 wt %, 1.5 wt %, 2.0 wt %, 2.5 wt %, 3.0 wt %, 3.5 wt %, 4.0 wt %, 4.5 wt % or more, and/or 9.5 wt %, 9.0 wt %, 8.5 wt %, 8.0 wt %, 7.5 wt %, 7.0 wt %, 6.5 wt %, 6.0 wt %, 5.5 wt %, 5.0 wt % or less, about 0.5 wt % to about 8.0 wt %, or about 3.0 wt % to about 5.0 wt %. The content analysis of polyol in the disclosed product is performed by first hydrolyzing the product with hyaluronidase, and then analyzing the content by HPLC according to the method described in the Ph. Eur. 10.0.

In certain embodiments, the crosslinked HA gel further comprises linear HA. Linear HA is a polysaccharide, in which the β-1,4 bonds between β-1,3-N-acetyl glucosamine and β-1,4-glucuronic and the β-1,3 bonds between the repeating disaccharide units formed of β-1,3-N-acetyl glucosamine and β-1,4-glucuronic are all linear bonds. For the crosslinked HA gel of the present disclosure, in order to extend its utility as an injectable product when filled into a syringe-namely, to reduce the injection force required during use, or to meet specific viscoelastic property requirements of the product - linear HA may be mixed into the product. The amount of linear HA added is not limited; however, it is required that the final product of the crosslinked HA gel has a modulus of elasticity (G') equaling or exceeding its modulus of viscosity (G'') when analyzed by a rheometer at a temperature of 25 °C and a frequency of 0.1 Hz to 10.0 Hz.

In certain embodiments, the crosslinked HA gel consists of the crosslinked hyaluronic acid or the metal salt thereof, triamcinolone or the pharmaceutical acceptable salt or ester thereof, optionally a polyol, optionally a linear hyaluronic acid, and optionally an auto-crosslinked hyaluronic acid.

In some embodiments, the crosslinked HA gel comprises auto-crosslinked HA. Auto-crosslinked HA is an ester formed through an esterification reaction between the hydroxyl groups (-OH) and carboxyl groups (-COOH) of HA molecules, either intermolecularly or intramolecularly, in the presence of a crosslinking agent. Since no crosslinking agent is used, there are no molecular fragments of crosslinking groups present in the resulting auto-crosslinked HA, allowing it to retain the excellent biocompatibility and viscoelastic properties of HA. As a result, it has been widely used in various medical applications. For the crosslinked HA gel of the present disclosure, in order to extend utility thereof as an injectable product when filled into a syringe, namely, to reduce the injection force required during use through reducing the viscosity of the product, or to meet specific viscoelastic property requirements of the product, auto-crosslinked HA may be mixed into the product. Auto-crosslinked HA can be formed under low temperature and acidic conditions, for example, by the method disclosed in Taiwan Patent No. TWI840941, or by the method disclosed in U.S. Patent No. 5,676,964. While the amount of auto-crosslinked HA added and HA content thereof are not limited, it is required that the final product of the crosslinked HA gel have a modulus of elasticity (G') equaling or exceeding modulus of viscosity (G'') when analyzed by a rheometer at a temperature of 25 °C and a frequency of 0.1 Hz to 10.0 Hz. In an embodiment of the present disclosure, the content of auto-crosslinked HA of the crosslinked HA gel may be about 0 wt % to about 50 wt %, about 5 wt % to about 50 wt %, about 5 wt % to about 40 wt %, about 5 wt % to about 30 wt %, or about 10 wt % to about 30 wt %. In another embodiment of the present disclosure, the HA content of the auto-crosslinked HA may be about 1.5 wt % to about 7.5 wt %, about 2.0 wt % to about 6.0 wt %, about 2.5 wt % to about 5.0 wt %, about 1.0 wt %, about 2.0 wt %, or about 3.0 wt %.

In some embodiments, the pH value of the crosslinked HA gel disclosed herein may be about pH 6.5 to 7.8, or about pH 6.5 to 7.5, to meet the requirements for medical applications. Additionally, the osmotic pressure of normal synovial fluid is approximately 404 ± 57 mOsm/kg. Therefore, in some embodiments, the osmotic pressure of the crosslinked HA gel of the present disclosure may be about 250 mOsm/kg to about 400 mOsm/kg, or about 300 mOsm/kg to about 350 mOsm/kg, to be suitable for use as intra-articular injection for human. The aforementioned osmotic pressure may be adjusted using salts (e.g., sodium chloride).

In some embodiments, the crosslinked HA gel disclosed herein is preferably stored in a light-protected environment to maintain the stability of triamcinolone or the pharmaceutically acceptable salt or ester thereof. After storage at 50°C for 9 months (equivalent to storage at 30°C for 36 months), the content of triamcinolone or the pharmaceutically acceptable salt or ester thereof in the crosslinked HA gel of the present disclosure can retain 90% or more of its initial content before storage, and the total impurity content (including impurity B, impurity C, and all unknown impurities) is less than 1.0 wt %, less than 0.5 wt %, or less than 0.3 wt % of the content of triamcinolone or the pharmaceutically acceptable salt or ester thereof. The analysis of total impurity content may be performed by initially hydrolyzing the product with hyaluronidase, followed by analysis using HPLC in accordance with the method described in the Ph. Eur. 10.0.

Furthermore, under light-protected conditions, after storage at 50°C for 9 months, the crosslinked HA gel still exhibits good viscoelastic properties. The modulus of elasticity (G') shows a decrease of less than 40%, less than 30%, or less than 20% compared to the initial value (i.e., at month 0).

The following examples are given for the purpose of illustration only and are not intended to limit the scope of the present invention.

### Example 1: Two stage mixing, reaction concentration: HA 12.7 wt %, BDDE 0.29 wt %, NaOH 0.82 wt %

Ten grams (10 g) of HA powder (intrinsic viscosity 2.1 $m^3$/kg, average molecular weight 1,289,000, water content 3.8%) and 2.2 g of triamcinolone hexacetonide (TH) powder (from Farmabios, purity 99.7%, water content 0.05%, micronized particles with an average particle size of 2.90 $\mu$m) are mixed. Then, 26 g of deionized water is added, and the mixture is stirred at 253 rpm for 2 minutes. Next, 34 g of deionized water, 3.1 g of 5 N sodium hydroxide aqueous solution, and 0.198 mL of BDDE (density 1.1 g/$cm^3$) are added, and the mixture is stirred for 5 minutes. The mixture is then allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 4 minutes using a homogenizer. Three grams (3 g) of the homogenized colloid product are filled into a 3 mL glass syringe and steam sterilized at 121 °C for 15 minutes to form the final product.

### TH Content in the Product

Mobile phase: water:methanol = 25:75 (v/v, HPLC grade)
Column: SUPELCO, SUPELCOSIL™ LC-18-DB, 25 cm × 7.8 mm, 5 $\mu$m
Detector: a spectrophotometer at a wavelength of 254 nm

Preparation of standard solution: 25 mg of TH chemical reference substance (CRS) is added into a volumetric flask, diluted to 10 mL with methanol and mixed thoroughly. Next, 1 mL of the mixture solution is taken and further diluted to 5 mL, and then filtered through a syringe filter (pore size 0.2 $\mu$m).

Preparation of sample: 1 g of gel sample is added into a 20 mL sample vial with 2 mL of 1000 U/mL hyaluronidase (Merck, Hyaluronidase, Product No. H1115000) aqueous solution, and then incubated in a 37 °C water bath for 16 hours until the sample is completely degraded. Next, 9 mL of methanol is added and mixed thoroughly, and then filtered through a syringe filter (pore size 0.2 $\mu$m).

The standard solution and sample are injected into the HPLC, with analysis conditions: mobile phase flow rate of 2.0 mL/min. The TH content in the sample and the total impurity content of TH in the sample are calculated according to the method described in the Ph. Eur. 10.0.

### Example 2: Two stage mixing, reaction concentration: HA 11.2 wt %, BDDE 0.25 wt %, NaOH 0.72 wt %

Ten grams (10 g) of HA powder (intrinsic viscosity 2.1 $m^3$/kg, average molecular weight 1,289,000, water content 3.8%) and 12.5 g of TH powder (from Farmabios, purity 99.7%, water content 0.05%, micronized particles with an average particle size of 2.90 $\mu$m) are mixed. Then, 26 g of deionized water is added, and the mixture is stirred at 253 rpm for 2 minutes. Next, 34 g of deionized water, 3.1 g of 5 N sodium hydroxide aqueous solution, and 0.198 mL of BDDE (density 1.1 g/$cm^3$) are added, and the mixture is stirred for 5 minutes. The mixture is then allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 4 minutes using a homogenizer. Three grams (3 g) of the homogenized colloid product are filled into a 3 mL glass syringe and steam sterilized at 121 °C for 15 minutes to form the final product.

### Example 3: Two stage mixing, reaction concentration: HA 12.7 wt %, BDDE 0.29 wt %, NaOH 0.82 wt %

Ten grams (10 g) of HA powder (intrinsic viscosity 2.1 $m^3$/kg, average molecular weight 1,289,000, water content 3.8%) and 12.5 g of TH powder (from Farmabios, purity 99.7%, water content 0.05%, micronized particles with an average particle size of 2.90 $\mu$m) are mixed. Then, 26 g of deionized water is added, and the mixture is stirred at 253 rpm for 2 minutes. Next, 23.7 g of deionized water, 3.1 g of 5 N sodium hydroxide aqueous solution, and 0.198 mL of BDDE (density 1.1 g/cm$^3$) are added, and the mixture is stirred for 5 minutes. The mixture is then allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 4 minutes using a homogenizer. Three grams (3 g) of the homogenized colloid product are filled into a 3 mL glass syringe and steam sterilized at 121 °C for 15 minutes to form the final product.

**Example 4: Containing mannitol, two stage mixing, reaction concentration: HA 12.7 wt %, BDDE 0.29 wt %, NaOH 0.82 wt %**

Ten grams (10 g) of HA powder (intrinsic viscosity 2.1 m$^3$/kg, average molecular weight 1,289,000, water content 3.8%) and 2.2 g of TH powder (from Farmabios, purity 99.7%, water content 0.05%, micronized particles with an average particle size of 2.90 $\mu$m) are mixed. Then, 26 g of deionized water is added, and the mixture is stirred at 253 rpm for 2 minutes. Next, 34 g of deionized water, 3.1 g of 5 N sodium hydroxide aqueous solution, and 0.198 mL of BDDE (density 1.1 g/cm$^3$) are added, and the mixture is stirred for 5 minutes. The mixture is then allowed to react at 20 °C for 7 days to form a colloid.

Twenty four grams (24.0 g) of mannitol is added into 376 g of a pH 7.0, 4 mM sodium phosphate buffer solution, and stirred until completely dissolved. The crushed colloid is then added into the mixture, and stirred until the colloid absorbs the buffer and swells. The colloid is adjusted with 6 N hydrochloric acid to pH 6.8, and then homogenized at 8,000 rpm for 4 minutes using a homogenizer. Three grams (3 g) of the homogenized colloid product are filled into a 3 mL glass syringe and steam sterilized at 121 °C for 15 minutes to form the final product.

**Mannitol Content in the Product**

Mobile phase: water (HPLC grade)
Column: Benson, BP-800 ca, 300 × 7.8 mm

Preparation of standard solution: 500 mg of mannitol (EP standard) is added into and diluted with the mobile phase solution to 100 mL, and is stirred until dissolved. Then, 500 mg of this solution is taken and further diluted with the mobile phase solution to 10 mL, and then filtered through a syringe filter (pore size 0.2 $\mu$m).

Preparation of sample: 0.5 g of sample is added into 2 mL of 1000 U/mL hyaluronidase (Merck, Hyaluronidase, Product No. H1115000) aqueous solution, and then incubated in a 37 °C water bath for 18 hours. Next, 7.5 mL of the mobile phase solution is added to the incubated product and mixed thoroughly, and then filtered through a syringe filter (pore size 0.2 $\mu$m).

The standard solution and sample are injected into the HPLC, with analysis conditions: mobile phase flow rate of 0.5 mL/min, column temperature 80 °C, refractive index detector temperature 40 °C. The mannitol content in the sample is calculated according to the method described in the Ph. Eur. 10.0.

**Example 5 (preparation of sample for stability test): Two stage mixing and containing linear HA, reaction concentration: HA 12.60 wt %, BDDE 0.29 wt %, NaOH 0.81 wt %**

Ten grams (10 g) of HA powder (intrinsic viscosity 2.1 m$^3$/kg, average molecular weight 1,289,000, water content 3.8%) and 3.1 g of TH powder (from Farmabios, purity 99.7%, water content 0.05%, micronized particles with an average particle size of 2.90 $\mu$m) are mixed. Then, 26 g of deionized water is added, and the mixture is stirred at 253 rpm for 2 minutes. Next, 34 g of deionized water, 3.1 g of 5 N sodium hydroxide aqueous solution, and 0.198 mL of BDDE (density 1.1 g/cm$^3$) are added, and the mixture is stirred for 5 minutes. The mixture is then allowed to react at 20 °C for 7 days to form a colloid. The colloid is first crushed, then 370 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 4 minutes using a homogenizer. Then, 50 g of 2 wt % linear HA aqueous solution is added, and the mixture is again homogenized at 8,000 rpm for 4 minutes. Three grams (3 g) of the homogenized colloid product are filled into a 3 mL glass syringe and steam sterilized at 121 °C for 15 minutes to form the final product. The average particle size of the product is analyzed using a laser particle size analyzer (PARTICA LA-960, HORIBA) and found to be 418.57 ± 4.81 $\mu$m.

**Comparative Example 1: One stage mixing, reaction concentration: HA 12.7 wt %, BDDE 0.29 wt %, NaOH 0.82 wt %**

Ten grams (10 g) of HA powder (intrinsic viscosity 2.1 m$^3$/kg, average molecular weight 1,289,000, water content 3.8%), 2.2 g of TH powder (from Farmabios, purity 99.7%, water content 0.05%, micronized particles with an average particle size of 2.90 $\mu$m), 60 g of deionized water, 3.1 g of 5 N sodium hydroxide aqueous solution and 0.198 mL of BDDE (density 1.1 g/cm$^3$) are mixed and stirred at 253 rpm for 7 minutes, and then the mixture is allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 2 minutes using a homogenizer. Three grams (3 g) of the homogenized colloid product are filled into a 3 mL glass syringe and steam sterilized at 121 °C for 15 minutes to form the final product.

**Comparative Example 2: One stage mixing, TH added after crosslinking reaction and during neutralization and homogenization, reaction concentration: HA 13.1 wt %, BDDE 0.30 wt %, NaOH 0.85 wt %**

Ten grams (10 g) of HA powder, 60 g of deionized water, 3.1 g of 5 N sodium hydroxide aqueous solution and 0.198 mL of BDDE (density 1.1 g/cm$^3$) are mixed and stirred at 253 rpm for 7 minutes, and then the mixture is allowed to react at 20 °C for 7 days to form a colloid. The colloid is first crushed, then 2.2 g of TH powder (from Farmabios, purity 99.7%, water content 0.05%, micronized particles with an average particle size of 2.90 $\mu$m) and 400 g of a pH 7.0, 4 mM sodium phosphate buffer solution containing 0.66 wt % sodium chloride are added, and the mixture is stirred until the colloid absorbs the buffer and swells. The colloid is adjusted with 6 N hydrochloric acid to pH 6.8, and then homogenized at 8,000 rpm for 4 minutes using a homogenizer. Three grams (3 g) of the homogenized colloid product are filled into a 3 mL glass syringe and steam sterilized at 121 °C for 15 minutes to form the final product.

**Testing Method for Sustained Release of TH of Gel**

Three grams (3.0 g) of the gel sample are placed in a stainless steel mesh container (standard basket for dissolution test according to the United States Pharmacopeia USP711, with a height of 30.7 $\pm$ 3.0 mm, inner diameter of 20.2 $\pm$ 1.0 mm, outer diameter of 22.2 $\pm$ 1.0 mm, and stainless steel mesh aperture of 100 mesh). The container is placed into a 60 mL sample vial containing a magnetic stir bar, and 35 mL of 20 U/mL hyaluronidase aqueous solution is added into the sample vial. The sample vial is incubated in a 37°C water bath and stirred continuously at 350 rpm with the magnetic stir bar located beneath the mesh container.

At each reaction time point (0, 1, 2, 4, 6, and 8 hours), 1 mL of the hydrolysate outside the stainless steel mesh container is withdrawn for testing. The reaction endpoint is at 8 hours.

Analysis of TH Content: To each collected hydrolysate sample, 3 mL of methanol is added and mixed thoroughly. The sample is filtered through a 0.2 $\mu$m pore size syringe filter, and the TH content is analyzed by HPLC.

$$\text{TH release \%} = \frac{\text{TH content of hydrolaysate at each reaction time point}}{\text{TH content of hydrolaysate at 8 hours}} \text{ X } 100\%$$

**Table 1: HA content, mannitol content and viscoelastic properties of the Examples, Comparative Examples, and a commercial product.**

|  | HA (wt %) | Mannitol (wt %) | G'(Pa) (25 °C, 1Hz) | G"(Pa) (25 °C, 1Hz) | Average diameter* ($\mu$m) |
|---|---|---|---|---|---|
| Example 1 | 2.02 | - | 456.6 | 56.3 | 405.95$\pm$4.22 |
| Example 2 | 2.07 | - | 430.9 | 45.6 | 733.03$\pm$4.71 |
| Example 3 | 2.12 |  | 1127.0 | 96.1 | 759.61$\pm$9.81 |
| Example 4 | 2.03 | 4.92 | 711.3 | 77.0 | 490.46$\pm$10.38 |
| Comparative Example 1 | 2.03 | - | 440.8 | 35.4 | 437.02$\pm$16.97 |
| Comparative Example 2 | 2.12 | - | 429.7 | 40.6 | 444.41$\pm$5.15 |

(continued)

|  | HA (wt %) | Mannitol (wt %) | G'(Pa) (25 °C, 1Hz) | G"(Pa) (25 °C, 1Hz) | Average diameter* (μm) |
|---|---|---|---|---|---|
| Cingal® | 2.03 | - | 103.2 | 95.7 | 28.79±15.5 |

*Each value is presented as mean ± standard deviation (n = 5).

**Table 2: TH content of the Examples and Comparative Examples.***

|  | Example 1 | Example 2 | Example 3 | Example 4 | Comparative Example 1 | Comparative Example 2 |
|---|---|---|---|---|---|---|
| TH % | 0.47 | 2.57 | 2.66 | 0.47 | 0.46 | 0.44 |
| RSD (%)** | 1.22 | 1.28 | 1.27 | 0.80 | 6.29 | 9.83 |

*Total impurity content of TH is less than 0.05% for each of the Examples and Comparative Examples.
**The analysis of RSD (%) includes 9 samples, with each measured 3 times.

**Table 3: TH release % of the gels of the Examples and the commercial product.**

| Reaction time (hr) | Example 1 | Example 2 | Comparative Example 1 | Cingal® |
|---|---|---|---|---|
| 0 | 0.0% | 0.0% | 0.0% | 0.0% |
| 1 | 20.97% | 15.55% | 36.23% | 86.55% |
| 2 | 34.12% | 30.13% | 52.57% | 92.02% |
| 4 | 74.47% | 68.25% | 85.14% | 94.71% |
| 6 | 86.00% | 81.17% | 94.16% | 95.83% |
| 8 | 100.00% | 100.00% | 100.00% | 100.00% |

**Table 4: Properties of the product stored under light-protected condition and at 50 °C for different durations.**

|  | The 0th month | The 1st month | The 3rd month | The 6th month | The 9th month |
|---|---|---|---|---|---|
| HA content (wt %) | 2.12 | 2.11 | 2.15 | 2.15 | 2.12 |
| pH value | 7.25 | 7.25 | 7.30 | 7.29 | 7.30 |
| Osmotic pressure (mOsm/kg) | 304 | 304 | 311 | 303 | 306 |
| Modulus of elasticity G'(Pa) measured at 25 °C, 1 Hz | 267.9 | 235.6 | 232.0 | 223.1 | 220.5 |
| Modulus of viscosity G" (Pa) measured at 25 °C, 1 Hz | 43.6 | 35.9 | 41.7 | 41.3 | 35.0 |
| TH content (%) | 0.63 | 0.63 | 0.65 | 0.65 | 0.65 |
| Total impurity content of TH (%) | < 0.05% | <0.05% | < 0.05% | < 0.05% | < 0.05% |

**Example 6: Preparation of Crosslinked HA Gel Products Containing 0 wt %, 10 wt %, and 30 wt % Auto-cross-linked HA**

Preparation of Auto-crosslinked HA Colloid: Auto-crosslinked hyaluronic acid is synthesized according to the method disclosed in Taiwan Patent TWI 840941. The reaction conditions are as follows: HA concentration at 10.0 wt % (intrinsic viscosity 1.7 $m^3$/kg, molecular weight 983,000), HCl concentration at 1.0 N, reaction temperature at -20 °C, and reaction time of 8 days. After homogenization, washing, and neutralization steps, a granular auto-crosslinked HA colloid with pH 7.0 is obtained. The average particle size suspended in physiological saline is measured by a laser particle size analyzer (PARTICA LA-960, HORIBA®) and found to be 295.42 ± 9.76 μm. The colloid is heated with steam at 121 °C for 15 minutes to form the auto-crosslinked HA gel.

Preparation of Crosslinked HA Colloid Containing TH (HA concentration 12.6 wt %, BDDE concentration 0.29 wt %, NaOH concentration 0.68 wt %): The reaction was conducted using the two-stage mixing method disclosed herein. First, 10 g of HA powder (intrinsic viscosity 2.1 $m^3$/kg, average molecular weight 1,289,000, water content 3.8%) and 3.24 g of (TH) powder (from Farmabios, purity 99.7%, water content 0.05%, micronized particles with an average particle size of 2.90 $\mu$m) are mixed thoroughly. Then, 26 g of deionized water was added and stirred at 253 rpm for 2 minutes. Subsequently, 34 g of deionized water, 2.6 g of 5 N sodium hydroxide aqueous solution, and 0.198 mL of BDDE (density 1.1 g/cm$^3$) are added, and the mixture is stirred for 5 minutes. The mixture is then allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 5 minutes using a homogenizer to form the non-sterile TH-containing crosslinked HA colloid. Three grams (3 g) of the homogenized colloid are filled into a 3 mL glass syringe and steam sterilized at 121 °C for 15 minutes to obtain the TH-containing gel product without auto-crosslinked HA. The average particle size is analyzed by laser particle size analyzer (PARTICA LA-960, HORIBA®), with the gel product suspended in physiological saline.

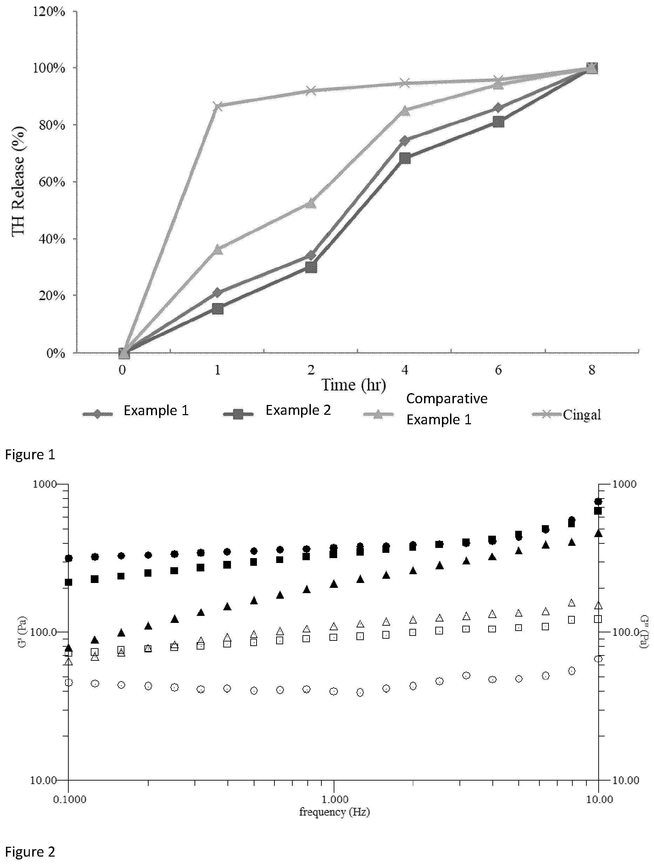

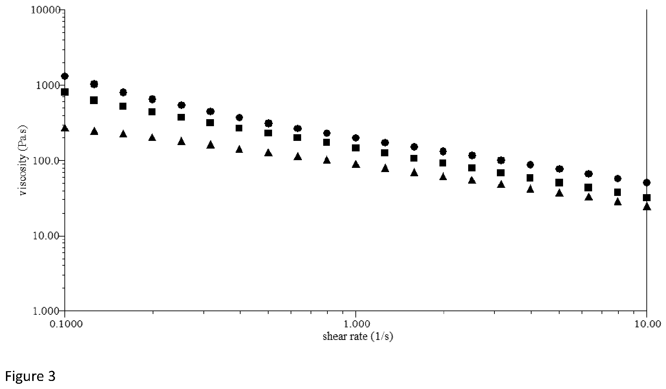

The pH 7.0 granular auto-crosslinked HA colloid and the TH-containing crosslinked HA colloid are mixed thoroughly at weight ratios of 10:90 (containing 10 wt % auto-crosslinked HA) and 30:70 (containing 30 wt % auto-crosslinked HA) prior to steam sterilization. Three grams (3g) of each mixture are filled into 3 mL glass syringes and sterilized by steam autoclaving at 121 °C for 15 minutes, respectively, forming TH-containing crosslinked HA gels containing 10 wt % and 30 wt % auto-crosslinked HA. Their properties are shown in Table 5 and Figures 2 and 3.

### Table 5: Properties of the products of Example 6

|  | Auto-crosslinked colloid HA content | | | |
|---|---|---|---|---|
|  | 100 wt % | 0 wt % | 10 wt % | 30 wt % |
| HA content (wt %) | 2.57 | 2.06 | 2.10 | 2.15 |
| pH value | 6.80 | 6.94 | 6.93 | 6.89 |
| Osmotic pressure (mOsm/kg) | 58 | 334 | 303 | 266 |
| Average diameter* ($\mu$m) | - | 258.04$\pm$8.89 | 240.84$\pm$7.34 | 263.40$\pm$4.22 |
| Modulus of elasticity G'(Pa) measured at 25 °C, 1 Hz | 139.2 | 369.8 | 335.4 | 213.0 |
| Modulus of viscosity G" (Pa) measured at 25 °C, 1 Hz | 108.1 | 39.76 | 91.79 | 110.0 |
| Shear viscosity $\mu$ (Pa.s) measured at 25 °C, 1 S$^{-1}$ | 67.12 | 194.9 | 145.8 | 90.43 |
| TH content (%) | - | 0.68 | 0.61 | 0.47 |
| Total impurity content of TH (%) | - | < 0.05% | < 0.05% | <0.05% |

*Each value is presented as mean $\pm$ standard deviation (n = 5).

### Example 7: Degree of Cross-linking (CrD) of the Crosslinked HA Gel

Testing method of the degree of cross-linking is based on the methods used by Frida J. Wende et al. ("1D NMR Methods for Determination of Degree of Cross-linking and BDDE Substitution Positions in HA Hydrogels," Carbohydrate Polymers, Volume 157, February 2017, Pages 1525-1530) and Lennart Kenne et al. ("Modification and Cross-linking Parameters in Hyaluronic Acid Hydrogels- Definitions and Analytical Methods," Carbohydrate Polymers, Volume 1, January 2013, Pages 410-418). The testing method involves taking 3 g of gel sample and washing it with 60 mL of physiological saline solution (9 mg/mL sodium chloride), followed by filtration to remove the filtrate. This washing and filtration step is repeated three times. Then, 60 mL of pure water is added for washing, followed by filtration to remove the filtrate; this step is also repeated three times. Subsequently, 10 mL of 1 mM phosphate buffer solution (pH 7.0) and 0.3 mL of 4 U/mL chondroitinase ABC (chABC) enzyme solution are added to the sample, and enzymatic hydrolysis is carried out at 37°C for 24 hours with continuous stirring. After complete hydrolysis of the gel, centrifugation (3000 rpm, 15 minutes) is performed to remove the lower precipitate of TH powder, and the supernatant is collected for lyophilization. Prior to NMR analysis, the lyophilized sample is re-dissolved in 0.25 mL of D$_2$O and transferred to an NMR tube for carbon-13 ($^{13}$C) NMR analysis using a 500 MHz NMR spectrometer (Bruker UltraShield Plus (500 MHz)).

NMR equipment specifications and detection parameters are as follows.

| Temperature | 298 K |
|---|---|
| Pulse Sequence | zgig30 |
| Probe | QNP ($^1$H/$^{13}$C/$^{31}$P/$^{19}$F) 5 mm CryoProbe with Z gradient |

(continued)

| Number of Scans | 2560 |
|---|---|
| Receiver Gain | 101.0 |
| Relaxation Delay | 15.0 s |
| Pulse Angle | 90° |
| System | NEO 500, four-channel system for liquid state NMR |

The degree of cross-linking (CrD) can be calculated by the following formulae.

$$MoD\ (\%) = (I^{\delta C24\text{-}26} / 2) / I^{\delta C21\text{-}23} \times 100\% \qquad (1)$$

$$CrR = 1 - I^{\delta C62\text{-}63} / (I^{\delta C24\text{-}26} / 2) \qquad (2)$$

$$CrD\ (\%) = CrR \times MoD \qquad (3)$$

Where:

I: Integral value of the carbon-13 ($^{13}$C) NMR spectrum
MoD: Degree of modification, representing the molar percentage of all crosslinking agent BDDE molecules relative to HA in the product, including both mono- and double-linked BDDE
CrR: Effective cross-linker ratio, representing the molar ratio of double-linked BDDE to all BDDE molecules in the product
CrD: Degree of cross-linking, representing the percentage of double-linked in the product

**Preparation**

Sample Preparation for Degree of Cross-linking Testing: Products with different viscoelasticity can be obtained by adjusting the amount of base added during the crosslinking reaction (i.e., changing the reaction concentration of the base). First, 10 g of HA powder (intrinsic viscosity 2.395 m$^3$/kg, average molecular weight 1,525,000, water content 5.1%) is mixed thoroughly with 3.4 g of TH powder (from Farmabios, purity 99.7%, water content 0.05%, micronized particles with an average particle size of 2.90 μm). Then, 26 g of deionized water is added, and the mixture is stirred at 253 rpm for 2 minutes. Next, 34 g of deionized water, 0.198 mL of BDDE (density 1.1 g/cm$^3$), and different amounts of 5 N sodium hydroxide aqueous solution (Product 1: 2.1 g; Product 2: 2.7 g; Product 3: 3.8 g, with high to low viscoelastic properties) are added, and the mixture is stirred for 7 minutes. The mixture is then allowed to react at 20 °C for 7 days to form a colloid. The colloid is first crushed, then 400 g of a pH 7.0, 4 mM sodium phosphate buffer solution containing 0.66 wt % sodium chloride is added, and the mixture is stirred until the colloid absorbs the buffer and swells. The colloid is adjusted with 6 N hydrochloric acid to pH 6.8, and then homogenized at 8,000 rpm for 4 minutes using a homogenizer.

The homogenized colloid is mixed thoroughly with a 2 wt % hyaluronic acid solution (HA powder with intrinsic viscosity 2.395 m$^3$/kg, dissolved in 4 mM sodium phosphate buffer at pH 7.0) at a weight ratio of 9:1. Finally, 3 g of the mixture is filled into a 3 mL glass syringes and steam sterilized at 121 °C for 15 minutes to form the final product.

**Table 6: Properties and degree of cross-linking of Products 1 to 3**

| | Product 1 | Product 2 | Product 3 |
|---|---|---|---|
| Modulus of elasticity G'(Pa) measured at 25 °C, 1 Hz | 796.5 | 269.9 | 79.5 |
| Modulus of viscosity G" (Pa) measured at 25 °C, 1 Hz | 133.5 | 51.9 | 20.1 |
| HA content (wt %) | 2.09 | 2.03 | 2.08 |
| pH value | 6.98 | 6.93 | 6.89 |
| Osmotic pressure (mOsm/kg) | 288 | 289 | 289 |
| TH content (%) | 0.66 | 0.62 | 0.63 |
| Total impurity content of TH (%) | < 0.05 | < 0.05 | < 0.05 |

(continued)

|  | Product 1 | Product 2 | Product 3 |
|---|---|---|---|
| MoD(%)* | 2.800±0.163 | 2.752±0.061 | 2.092±0.083 |
| CrR* | 0.602±0.044 | 0.524±0.074 | 0.486±0.075 |
| CrD(%)* | 1.682±0.030 | 1.442±0.215 | 1.020±0.194 |
| *Each value is presented as mean ± standard deviation (n = 3). | | | |

While the present disclosure has been described and illustrated with reference to specific embodiments thereof, the descriptions and illustrations are not limiting. It should be understood by those skilled in the art that various changes may be made and equivalents may be substituted without departing from the true spirit and scope of the present disclosure as defined by the appended claims.

**Claims**

1.  A method for manufacturing a crosslinked hyaluronic acid gel, comprising steps of:

    (a) mixing hyaluronic acid or a metal salt thereof and triamcinolone or a pharmaceutical acceptable salt or ester thereof to form a mixture;
    (b) adding water into the mixture of step (a) to form a colloid; and
    (c) crosslinking the colloid of step (b) with 1,4-butanediol diglycidyl ether (BDDE).

2.  The method of Claim 1, wherein triamcinolone or the pharmaceutical acceptable salt or ester thereof of step (a) is triamcinolone acetonide 21-oic acid methyl ester, triamcinolone benetonide, triamcinolone hexacetonide or triamcinolone diacetate.

3.  The method of Claim 1 or 2, wherein in step (a), a weight ratio of hyaluronic acid or the metal salt thereof to triamcinolone or the pharmaceutical acceptable salt or ester thereof is about 10:1 to about 10:15.

4.  The method of any one of the preceding claims, wherein in step (a), hyaluronic acid or the metal salt thereof and/or triamcinolone or the pharmaceutical acceptable salt or ester thereof is in solid form.

5.  The method of any one of the preceding claims, wherein in step (a), hyaluronic acid or the metal salt thereof has a water content of about 20% or less and/or a particle size of 200 $\mu$m or less.

6.  The method of any one of the preceding claims, wherein in step (a), triamcinolone or the pharmaceutical acceptable salt or ester thereof has a water content of about 2% or less and/or a particle size of 5 $\mu$m or less.

7.  The method of any one of the preceding claims, wherein in step (b), a weight ratio of hyaluronic acid or the metal salt thereof to water is about 1:1 to about 1:6.

8.  The method of any one of the preceding claims, wherein in step (c), a reaction concentration of hyaluronic acid or the metal salt thereof is about 10 wt % to about 30 wt %.

9.  The method of any one of the preceding claims, further comprising:
    (d) steam sterilizing the crosslinked product of step (c).

10. A crosslinked hyaluronic acid gel manufactured by the method of any one of Claims 1 to 9, wherein

    triamcinolone is substantially evenly dispersed in the crosslinked hyaluronic acid gel; and
    the crosslinked hyaluronic acid gel is free of polysorbate.

11. The crosslinked hyaluronic acid gel of Claim 10, which has an average particle diameter of about 150 $\mu$m to about 1000 $\mu$m.

12. The crosslinked hyaluronic acid gel of Claim 10 or 11, which has a modulus of elasticity (G') measured at 25 °C and 1 Hz of about 50 Pa to about 1500 Pa, and a modulus of viscosity (G'') measured at 25 °C and 1 Hz of about 10 Pa to about 150 Pa.

13. The crosslinked hyaluronic acid gel of any one of Claims 10 to 12, which has a degree of crosslinking (CrD) of about 0.05% to about 5.0%.

14. The crosslinked hyaluronic acid gel of any one of Claims 10 to 13, consisting of:

the crosslinked hyaluronic acid or the metal salt thereof;
triamcinolone or the pharmaceutical acceptable salt or ester thereof;
optionally a polyol;
optionally a linear hyaluronic acid; and
optionally an auto-crosslinked hyaluronic acid.

15. The crosslinked hyaluronic acid gel of any one of Claims 10 to 14 for use in the treatment of osteoarthritis.

Figure 1

Figure 2

Figure 3

**Europäisches Patentamt**
**European Patent Office**
**Office européen des brevets**

# EUROPEAN SEARCH REPORT

Application Number

EP 25 19 5007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2019/066505 A1 (LG CHEMICAL LTD [KR]) 4 April 2019 (2019-04-04) * the whole document * | 1-15 | INV. A61K9/06 A61K47/10 A61K47/36 A61K31/573 A61K31/58 A61P19/02 |
| Y | ASWATHY S H ET AL: "Commercial hydrogels for biomedical applications", HELIYON, vol. 6, no. 4, 1 April 2020 (2020-04-01), page e03719, XP093020813, ISSN: 2405-8440, DOI: 10.1016/j.heliyon.2020.e03719 * page 9; "4.4. Drug Delivery" * | 1-15 | |
| A | WO 2009/073508 A2 (ALLERGAN INC [US]) 11 June 2009 (2009-06-11) * page 1, lines 12-15 * * page 9, line 27 - page 10, line 20 * * page 14, line 14 - page 15, line 18 * * page 16, lines 15-23 * * page 18, line 1 - page 19, line 15 * * page 20, line 27 - page 23, line 23 * * claims 1-28 * | 1-15 | |

TECHNICAL FIELDS SEARCHED (IPC)

A61K
A61P

The present search report has been drawn up for all claims

| Place of search | Date of completion of the search | Examiner |
|---|---|---|
| The Hague | 7 January 2026 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 5007

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2026

| Patent document cited in search report | Publication date | Patent family member(s) | Publication date |
|---|---|---|---|
| WO 2019066505 A1 | 04-04-2019 | NONE | |
| WO 2009073508 A2 | 11-06-2009 | AU 2008331491 A1 | 11-06-2009 |
| | | BR PI0821080 A2 | 30-09-2014 |
| | | CA 2707060 A1 | 11-06-2009 |
| | | CN 101925347 A | 22-12-2010 |
| | | EP 2222714 A2 | 01-09-2010 |
| | | JP 2011505369 A | 24-02-2011 |
| | | KR 20100117058 A | 02-11-2010 |
| | | RU 2010125705 A | 10-01-2012 |
| | | US 2009143348 A1 | 04-06-2009 |
| | | US 2013136780 A1 | 30-05-2013 |
| | | WO 2009073508 A2 | 11-06-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9371402 B
- TW I840941
- US 5676964 A

**Non-patent literature cited in the description**

- **FRIDA J. WENDE et al.** 1D NMR Methods for Determination of Degree of Cross-linking and BDDE Substitution Positions in HA Hydrogels. *Carbohydrate Polymers*, February 2017, vol. 157, 1525-1530

- **LENNART KENNE et al.** Modification and Cross-linking Parameters in Hyaluronic Acid Hydrogels-Definitions and Analytical Methods. *Carbohydrate Polymers*, 01 January 2013, 410-418